(19) Europäisches Patentamt
European Patent Office
Office européen des brevets

(11) Numéro de publication : **0 200 840**
**B1**

(12) **FASCICULE DE BREVET EUROPÉEN**

(45) Date de publication du fascicule du brevet : **05.04.89**

(21) Numéro de dépôt : **85401868.6**

(22) Date de dépôt : **25.09.85**

(51) Int. Cl.$^4$ : **C 07 C 65/11**, C 07 C 65/24, C 07 C 51/367, C 07 C 51/09, C 07 C 69/94, C 07 C 67/39

(54) **Procédé de préparation d'acides 6-alkoxy(inférieur)-1-naphtoiques, éventuellement halogénés en 5 et des esters correspondants.**

(30) Priorité : **10.05.85 FR 8507107**

(43) Date de publication de la demande : **12.11.86 Bulletin 86/46**

(45) Mention de la délivrance du brevet : **05.04.89 Bulletin 89/14**

(84) Etats contractants désignés : **AT BE CH DE GB IT LI LU NL SE**

(56) Documents cités :
**US-A- 4 215 065**
**US-A- 4 439 617**
**US-A- 4 499 310**
**JOURNAL OF THE AMERICAN CHEMICAL SOCIETY, vol. 69, no. 10, 31 octobre 1947, pages 2261-2264; C.C. PRICE et al.: "Some naphthalene analogs of desoxy-corticosterone"**

(73) Titulaire : **DELALANDE S.A.**
**32, rue Henri Regnault**
**F-92400 Courbevoie (FR)**

(72) Inventeur : **Adrian, Guy**
**56, rue de Dobeln**
**F-69700 Givors (FR)**
Inventeur : **Garnier, Sylvain**
**19, rue des Treilles Corbas**
**F-69800 St-Priest (FR)**

(74) Mandataire : **Kedinger, Jean-Paul et al**
**c/o Cabinet Malemont 42, avenue du Président Wilson**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue St-Denis, 75001 Paris, France

**Description**

La présente invention a pour objet un nouveau procédé de préparation d'acides 6-alkoxy (inférieur)-1-naphtoïques et des esters correspondants, ainsi que l'utilisation de ce procédé pour la préparation d'acides 6-alkoxy (inférieur)-5-halo-1-naphtoïques et des esters correspondants.

Les acides et esters de ce type constituent des intermédiaires de synthèse particulièrement utiles pour la préparation de divers composés inhibiteurs de l'aldose réductase trouvant leur application dans le traitement des complications du diabète. Ces intermédiaires, leur préparation et leur transformation en composés inhibiteurs de l'aldose réductase, sont décrits notamment dans J.A.C.S., 69, 2261 (1947), dans le brevet US 4 408 077 et dans la demande de brevet européen 00 59596.

L'article paru dans J.A.C.S., 69, 2261 (1947) décrit en particulier la préparation de l'acide 6-méthoxy-1-naphtoïque et de l'ester méthylique correspondant, par condensation de l'anisole avec l'acide furoïque en présence de chlorure d'aluminium, suivie d'une hydrolyse acide, de l'isolement de l'acide résultant et de l'estérification éventuelle par le méthanol de l'acide ainsi isolé. Comme il est précisé dans cet article, les conditions les plus satisfaisantes pour la préparation dudit acide consistent à opérer en présence d'un important excès d'anisole (10 équivalents) et en l'absence de tout autre solvant, avec une durée de réaction de 48 heures. La Demanderesse a constaté toutefois qu'en répétant le mode opératoire proposé dans l'article en cause, on parvenait à un mélange réactionnel pâteux difficilement agitable et, partant, difficile à homogénéiser et à hydrolyser, l'acide 6-méthoxy-1-naphtoïque n'étant obtenu après des opérations d'isolement fastidieuses, qu'avec des rendements très faibles (de l'ordre de 6 à 11 % par rapport à l'acide furoïque de départ) et sous la forme d'un produit impur. Tous ces inconvénients constituent donc des obstacles majeurs à la mise en œuvre industrielle du procédé proposé dans cet article.

L'un des buts de la présente invention est par conséquent de fournir un procédé de préparation d'acides 6-alkoxy(inférieur)-1-naphtoïques et des esters correspondants, avec des rendements améliorés, dans des conditions opératoires simples et de mise en œuvre industrielle aisée. La présente invention concerne plus précisément un procédé de préparation d'un composé de formule :

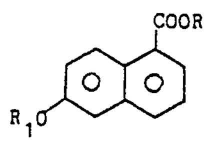

(I)

dans laquelle R = H ou alkyle inférieur et $R_1$ = alkyle inférieur, qui comprend la réaction, en présence d'un agent de condensation, de l'acide furoïque avec un composé de formule :

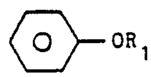

(II)

où $R_1$ a la même signification que ci-dessus, suivie d'une hydrolyse acide, ce qui conduit à un mélange de réaction comprenant deux composés respectivement de formule (III) et de formule (IV) :

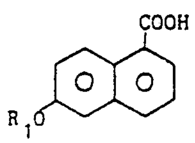

(III)

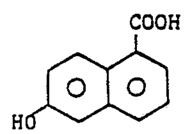

(IV)

où $R_1$ a la même signification que précédemment, ce procédé se caractérisant en ce qu'il comprend en outre l'action d'un agent alkylant sur les composés (III) et (IV) dudit mélange ou sur ces composés sous forme de sels, cet agent alkylant étant choisi pour former le composé de formule :

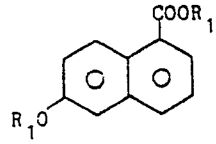

(Ia)

où $R_1$ a la même signification que précédemment et, si on le désire, l'hydrolyse du groupement ester du composé (Ia) pour obtenir l'acide correspondant de formule :

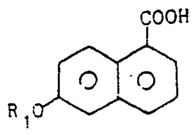

(Ib)

La Demanderesse a en effet mis en évidence pour la première fois que la réaction de condensation cyclisante entre l'acide furoïque et le composé (II) ne conduisait pas uniquement, après hydrolyse acide, à l'acide (III), mais en fait à un mélange contenant l'acide (III) et l'acide 6-hydroxy-1-naphtoïque (IV). Dans le procédé connu d'après l'article du J.A.C.S. précité, cet acide (IV) était purement et simplement éliminé au cours des opérations d'isolement et de purification de l'acide 6-méthoxy-1-naphtoïque, ce qui est l'une des raisons expliquant les faibles rendements avec lesquels est obtenu l'ester final.

Tout au contraire, plutôt que d'éliminer l'acide (IV), la Demanderesse a judicieusement exploité la présence de cet acide pour améliorer ces rendements, en soumettant le mélange des acides (III) et (IV) à l'action d'un agent alkylant choisi pour transformer le radical 6-hydroxy de l'acide (IV) en groupement alkoxy inférieur, ledit agent alkylant étant plus précisément choisi pour transformer le radical 6-hydroxy en un groupement $R_1O$ identique à celui présent en position 6 de l'acide (III). A nombre d'étapes égales, le procédé selon l'invention permet ainsi d'obtenir l'ester (Ia) avec des rendements largement supérieurs à ceux obtenus conformément au procédé connu et pouvant atteindre des valeurs de l'ordre de 30 % par rapport à l'acide furoïque de départ.

Il est à noter que l'agent alkylant peut être amené en réaction directement avec le mélange de réaction résultant de la condensation de l'acide furoïque et du composé (II) et de l'hydrolyse acide du produit de condensation résultant. On préfère toutefois que, préalablement à l'action dudit agent alkylant sur les composés (III) et (IV), ces derniers soient séparés du mélange de réaction. Cette séparation peut avantageusement être effectuée par extraction du mélange de réaction à un pH de 7-9 au moyen d'un agent basique en milieu aqueux, auquel cas les composés (III) et (IV) sont séparés sous forme de sels et amenés sous cette forme en réaction avec l'agent alkylant. L'agent basique peut par exemple être constitué par un hydrogénocarbonate de métal alcalin et en particulier par l'hydrogénocarbonate de potassium préféré en raison de sa grande solubilité dans l'eau.

L'agent alkylant peut être choisi dans le groupe comprenant les halogénures d'alkyle inférieur, les sulfates d'alkyle inférieur, les sulfates de di(alkyle inférieur) et leurs mélanges.

Cet agent alkylant est avantageusement mis en œuvre en milieu aqueux ou dans un milieu comprenant de l'eau et un solvant choisi parmi l'acétone, la méthyl éthyl cétone, le chlorure de méthylène et le dichloro-1,2 éthane, et en présence d'une base faible telle qu'une base minérale comme le carbonate de potassium par exemple.

L'agent de condensation utilisé pour la réaction de l'acide furoïque avec le composé (II), est généralement présent à raison de 3 à 5 moles par mole d'acide furoïque ; il est constitué par un acide de Lewis du type $AlCl_3$, une partie de cet acide pouvant être remplacée par un acide déshydratant tel que l'acide sulfurique, l'acide phosphorique, l'anhydride phosphorique et leurs mélanges.

La Demanderesse a par ailleurs mis en évidence que si la réaction de condensation de l'acide furoïque et du composé (II) était effectuée dans un solvant spécifique choisi dans le groupe comprenant : les hydrocarbures aliphatiques polyhalogénés tels que le tétrachloro-1,1,2,2 éthane, les hydrocarbures aromatiques polyhalogénés tels que l'ortho dichlorobenzène et leurs mélanges, il était possible de limiter de manière sensible la réaction parasite de déalkylation du composé (II) en phénol (réaction parasite très importante dans le procédé connu à la majeure partie de l'excès d'anisole est transformée en phénol) et, partant, d'augmenter le rendement de la réaction de condensation. Outre cet effet particulier, la présence de ce solvant permet d'opérer avec des quantités moindres de composé (II) (4 à 8 équivalents seulement par rapport à l'acide furoïque pour 10 équivalents dans le procédé connu), le mélange réactionnel est très fluide et donc facilement agitable et la durée de réaction est très écourtée puisque des durées de 6 à 10 heures permettent d'obtenir, dans les conditions définies ci-dessus, les composés (III) et (IV) avec des rendements globaux de l'ordre de 30 à 40 % par rapport à l'acide furoïque de départ.

Entre également dans la portée de la présente invention, l'application du procédé qui vient d'être décrit, à la préparation d'acides 6-alkoxy (inférieur)-5-halo-1-naphtoïques et des esters correspondants à partir de l'acide furoïque et de composés (II).

Par conséquent, la présente invention s'étend à un procédé de préparation d'un composé de formule :

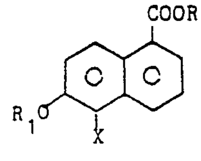

(V)

où R = H ou alkyle inférieur, $R_1$ = alkyle inférieur et X = halogène, consistant à préparer le composé de formule :

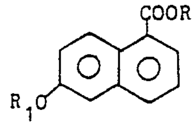

(I)

où $R_1$ et R ont la même signification que dans la formule (V), à partir de l'acide furoïque et du composé de formule :

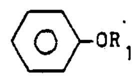

(II)

où $R_1$ = alkyle inférieure, puis à traiter le composé (I) ainsi obtenu, par un agent halogénant, ce procédé étant caractérisé en ce que la préparation du composé (I) est réalisée conformément au procédé défini dans la description qui précède à partir de l'acide furoïque et du composé (II).

L'halogénation du composé (I) peut être réalisée dans les conditions décrites par exemple dans la demande de brevet européen 00 59596 ou dans le brevet US 4 408 077, au moyen de chlore, de brome ou d'iode. Il est en outre possible d'effectuer cette réaction en milieu eau-hydrocarbure aliphatique halogéné tel que eau-dichloro-1,2 éthane, de préference à basse température pour éviter une polyhalogénation du noyau naphtalène, notamment à 0-30 °C.

Les composés halogénés (V) résultants sont ensuite isolés et purifiés de manière classique.

Les exemples ci-après sont donnés pour illustrer l'invention et visent en particulier la préparation de l'ester méthylique de l'acide 6-méthoxy 5-halo-1-naphtoïque.

## Exemple 1

Dans un réacteur de 1 litre, équipé d'un dispositif d'agitation à ancre tournante (350 tours/mn) on place 56 g (0,5 mole) d'acide furoïque, 270 g d'anisole (2,55 moles) et 187 ml d'orthodichlorobenzène.

Le mélange est chauffé à 40 °C et on introduit en 5 heures, 30 charges de 9 g de chlorure d'aluminium (2 moles), à raison d'une charge toutes les 10 minutes.

On chauffe à 80 °C pendant 3,5 heures, le milieu visqueux est ensuite dilué par 100 g d'anisole et 70 ml d'orthodichlorobenzène à 80 °C et agité pendant 1 heure puis hydrolysé par 1 500 ml d'acide chlorhydrique 6 N.

Entre 60 et 65 °C, on ajoute au milieu 250 ml d'acétate d'isopropyle et on laisse décanter pour obtenir 922 g d'une phase organique supérieure contenant, en poids, 2,61 % d'acide-6-méthoxy-1-naphtoïque et 1,30 % d'acide-6-hydroxy-1-naphtoïque.

Cette solution organique est extraite à 60 °C par 400 ml d'une solution saturée (350 g par litre) d'hydrogénocarbonate de potassium pour donner une phase aqueuse contenant les sels de ces deux acides naphtoïques. Ces acides sont libérés par de l'acide chlorhydrique à pH 1,2, extraits à 60 °C par 300 ml de dichloro-1,2 éthane et méthylés par 77,5 ml (0,82 mole) de sulfate de diméthyle en présence de 108 g (0,78 mole) de carbonate de potassium et de 500 ml d'acétone, entre 30 et 60 °C, pendant 3 heures.

Après élimination de l'acétone par distillation, le milieu réactionnel restant est lavé par deux fractions de 500 ml d'eau, puis traité par 67 g de brome (0,417 mole), à 25 °C, en présence de 100 ml d'eau.

Après 2 heures de réaction, la phase organique est séparée par décantation, puis concentrée sous vide à 50 °C ; l'addition au résidu d'un mélange de 130 ml d'acétate d'isopropyle et de 60 ml d'isopropanol, à 0 °C, permet de séparer par cristallisation puis séchage 40 g de 6-méthoxy-5-bromo-1-naphtoate de méthyle (point de fusion : 127 °C ; impureté CGL : 99-100 % ; rendement : 27 % par rapport à l'acide furoïque de départ).

## Exemple 2

On répète le mode opératoire de l'exemple 1, mais en remplaçant l'ortho dichlorobenzène par une quantité équivalente de tétrachloro-1,1,2,2 éthane.

On obtient finalement 38 g (rendement : 25,8 %) de 6-méthoxy-5-bromo-1-naphtoate de méthyle.

## Exemple 3

Comme dans l'exemple 1, on fait réagir 56 g d'acide furoïque et 270 g d'anisole, en présence de 187 ml d'ortho dichlorobenzène, à 40 °C, avec introduction de 20 charges de 9 g de chlorure d'aluminium (1,35 mole) à raison d'une charge toutes les 10 minutes, puis de 49 g d'acide sulfurique concentré (0,5 mole).

L'expérience est ensuite poursuivie comme dans l'exemple 1 pour conduire à 896 g d'une phase organique contenant 2,71 % en poids d'acide-6-méthoxy-1-naphtoïque et 0,77 % en poids d'acide 6-hydroxy-1-naphtoïque.

On obtient après méthylation et bromation effectuées comme dans l'exemple 1, 32,5 g de 6-méthoxy-5-bromo-1-naphtoate de méthyle (rendement : 22,5 %).

Exemple 4

Dans un réacteur de 6 $m^3$ on place 250 kg d'acide furoïque, 1 210 litres d'anisole et 835 litres d'ortho dichlorobenzène. On porte le mélange à 42 °C et tout en maintenant à cette température, on ajoute en 5,5 heures 1 200 kg de chlorure d'aluminium (33 charges de 36,36 kg à raison d'une charge toutes les 10 minutes).

La réaction est assez exothermique pendant la première moitié de l'introduction. On maintient 10 minutes à 42 °C, chauffe à 82 °C en 30 minutes et maintient cette température pendant 3 heures et 40 mn. On ajoute ensuite 445 litres d'anisole et 307 litres d'ortho dichlorobenzène.

On maintient 30 minutes à 82 °C, refroidit vers 65-70 °C et coule sur un mélange de 2 875 litres d'eau et 2 875 litres d'HCl 12,5 N sans dépasser 80 °C.

On rince le réacteur avec 1 000 litres d'acétate d'isopropyle, ajoute le mélange résultant au milieu d'hydrolyse, agite le tout 15 minutes à 65 °C puis décante. On élimine la phase aqueuse inférieure, conserve la phase organique supérieure (Poids ≃ 3 900 kg) qui contient ≃ 2,6 % d'acide 6-méthoxy-1-naphtoïque et ≃ 1,6 % d'acide 6-hydroxy-1-naphtoïque (rendement global en acides de 36-38 %). On soumet cette phase à trois extractions à 80 °C respectivement avec 1 340 litres, 223 litres et 223 litres d'une solution aqueuse saturée de $KHCO_3$ (à 400 g/1 100 ml) et on joint les phases aqueuses inférieures pour obtenir une solution aqueuse des sels de potassium des deux acides ci-dessus.

Dans un réacteur de 6 $m^3$, on charge 2 200 litres d'acétone, 520 kg de $K_2CO_3$ et 380 litres de $(CH_3)_2SO_4$.

On agite le mélange résultant et ajoute la solution aqueuse des sels de potassium obtenue précédemment. On chauffe le tout au reflux jusqu'à disparition desdits sels (vérification par CCM), résultat obtenu après environ 3 heures.

On évapore l'acétone à pression atmosphérique jusqu'à atteindre une vapeur à 100 °C. On refroidit le mélange restant à 60 °C et on y ajoute 2 000 litres d'eau, on extrait avec deux fractions de 500 litres de dichloro-1,2 éthane, on joint les phases dichloroéthaniques qui contiennent ≃ 125 kg de 6-méthoxy-1-naphtoate de méthyle (rendement ≃ 29 % par rapport à l'acide furoïque de départ). On introduit ensuite la solution dichloroéthanique (environ 1 300-1 400 litres) dans un réacteur de 3 $m^3$, on ajoute 350 litres d'eau, on agite à 20-25 °C et additionne 130 litres de brome (réaction exothermique) en maintenant le milieu à une température maximum de 25 °C. On agite pendant 1 heure après la fin de l'introduction du brome (détermination de la disparition de l'ester méthylique de l'acide 6-méthoxy-1-naphtoïque par CGL sur un échantillon de la phase organique inférieure).

On sépare la phase organique inférieure par décantation et la lave par 450 litres d'eau.

On sépare la phase organique lavée, évapore le solvant (sous vide en fin d'évaporation) et reprend le résidu par un mélange de 600 litres d'acétate d'isopropyle et 300 litres d'alcool isopropylique.

On chauffe jusqu'à dissolution complète vers 60 °C, puis refroidit et glace à 5 °C pendant 2 heures.

On sépare par filtration le précipité formé, on l'essore, on le lave par un mélange glacé de 50 litres d'acétate d'isopropyle et 100 litres d'alcool isopropylique et le sèche à 60 °C, ce qui conduit à 165-170 kg d'ester méthylique de l'acide 6-méthoxy-5-bromo-1-naphtoïque (rendement par rapport à l'acide furoïque de départ : 25 % environ).

Exemple 5

On effectue les réactions de condensation et de méthylation comme dans l'exemple 1 ; on fait ensuite réagir sur la solution brute de 6-méthoxy-1-naphtoate de méthyle obtenue 35,5 g de chlore (0,5 mole) à 20 °C en présence de 100 ml d'eau pendant 5 heures.

Après décantation, la phase organique est concentrée sous vide à 50 °C et le résidu est cristallisé dans 150 ml d'isopropanol, à 0 °C, pour donner 31,3 g de 6-méthoxy-5-chloro-1-naphtoate de méthyle qui est un composé nouveau.

Point de fusion : 125 °C.
Rendement par rapport à l'acide furoïque de départ : 25 %.
Spectre IR : 1 715 $cm^{-1}$ (COO).
Spectre RMN ($CDCl_3$), δ : 3,97 (s, 3H) ; 4,05 (s, 3H) ; 7,3-9,0 (m, 6H).

Exemple 6

On effectue les réactions de condensation et de méthylation comme dans l'exemple 1 ; on fait ensuite réagir sur la solution brute de 6-méthoxy-1-naphtoate de méthyle obtenue 53 g d'iode et 20 g d'acide iodique à 30 °C en présence de 200 ml d'acide acétique et de 200 ml d'une solution d'acide sulfurique N/2, pendant 5 heures.

Après décantation, la phase organique est lavée par une solution de 10 g de thiosulfate de sodium

dans 200 ml d'eau puis concentrée sous vide à 50 °C et le résidu est cristallisé dans 150 ml d'isopropanol, à 0 °C.

On obtient ainsi 36 g de 6-méthoxy-5-iodo-1-naphtoate de méthyle (point de fusion : 100 °C ; rendement par rapport à l'acide furoïque : 21 %).

**Revendications**

1. Procédé de préparation d'un composé de formule :

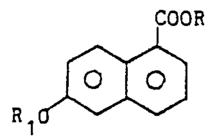

(I)

dans laquelle R = H ou alkyle inférieur et $R_1$ = alkyle inférieur, qui comprend la réaction, en présence d'un agent de condensation, de l'acide furoïque avec un composé de formule :

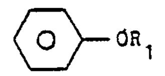

(II)

où $R_1$ a la même signification que ci-dessus, suivie d'une hydrolyse acide, ce qui conduit à un mélange de réaction comprenant deux composés respectivement de formule (III) et de formule (IV) :

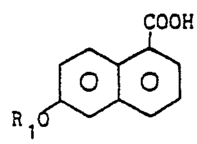

(III)

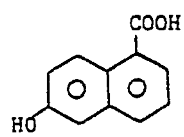

(IV)

où $R_1$ a la même signification que précédemment, caractérisé en ce qu'il comprend en outre l'action d'un agent alkylant sur les composés (III) et (IV) dudit mélange ou sur ces composés sous forme de sels, cet agent alkylant étant choisi pour former le composé de formule :

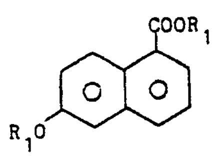

(Ia)

où $R_1$ a la même signification que précédemment et, si on le désire, l'hydrolyse du groupement ester du composé (Ia) pour obtenir l'acide correspondant de formule :

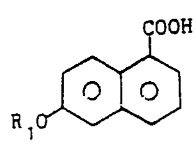

(Ib)

2. Procédé selon la revendication 1, caractérisé en ce que préalablement à l'action dudit agent alkylant sur les composés (III) et (IV), ces derniers sont séparés dudit mélange de réaction.

3. Procédé selon la revendication 2, caractérisé en ce que cette séparation est effectuée par extraction du mélange de réaction à un pH de 7-9 au moyen d'un agent basique en milieu aqueux.

4. Procédé selon la revendication 3, caractérisé en ce que l'agent basique est un hydrogénocarbonate de métal alcalin.

5. Procédé selon l'une quelconque des revendications précédentes, caractérisé en ce que l'agent alkylant est choisi dans le groupe comprenant les halogénures d'alkyle inférieur, les sulfates d'alkyle inférieur, les sulfates de di(alkyle inférieur) et leurs mélanges.

6. Procédé selon la revendication 5, caractérisé en ce que l'agent alkylant est mis en œuvre en milieu aqueux ou dans un milieu comprenant de l'eau et un solvant choisi parmi l'acétone, la méthyl éthyl cétone, le chlorure de méthylène et le dichloro-1,2 éthane.

7. Procédé selon l'une quelconque des revendications précédentes, caractérisé en ce que l'agent alkylant est utilisé en présence d'une base faible.

8. Procédé selon l'une quelconque des revendications précédentes, caractérisé en ce que l'agent de condensation est constitué par un mélange de chlorure d'aluminium et d'un acide déshydratant.

9. Procédé selon la revendication 8, caractérisé en ce que l'acide déshydratant est choisi dans le groupe comprenant l'acide sulfurique, l'acide phosphorique, l'anhydride phosphorique et leurs mélanges.

10. Procédé selon l'une quelconque des revendications précédentes, caractérisé en ce que la réaction de l'acide furoïque avec le composé (II) est effectuée en présence d'un solvant choisi dans le groupe comprenant : les hydrocarbures aliphatiques polyhalogénés, les hydrocarbures aromatiques polyhalogénés et leurs mélanges.

11. Procédé selon la revendication 10, caractérisé en ce que l'hydrocarbure aliphatique polyhalogéné est le tétrachloro-1,1,2,2 éthane et en ce que l'hydrocarbure aromatique polyhalogéné est l'ortho dichlorobenzène.

12. Procédé selon l'une quelconque des revendications précédentes pour la préparation des composés de formule (I) où $R_1$ = méthyle et R = H ou méthyle, caractérisé en ce que le composé de formule (II) est l'anisole et en ce que l'agent alkylant est un agent méthylant.

13. Procédé de préparation d'un composé de formule :

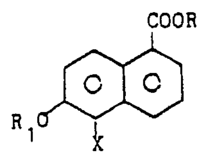

(V)

où R = H ou alkyle inférieur, $R_1$ = alkyle inférieur er X = halogène, consistant à préparer le composé de formule :

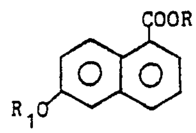

(I)

où $R_1$ et R ont la même signification que dans la formule (V), à partir de l'acide furoïque et du composé de formule :

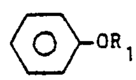

(II)

où $R_1$ = alkyle inférieur, puis à traiter le composé (I) ainsi obtenu, par un agent halogénant, caractérisé en ce que la préparation du composé (I) est effectuée conformément au procédé selon l'une quelconque des revendications 1 à 12.

14. Procédé selon la revendication 13, caractérisé en ce que le traitement du composé (I) par l'agent halogénant est effectué dans un milieu eau-hydrocarbure aliphatique halogéné.

**Claims**

1. A process for preparing a compound of formula :

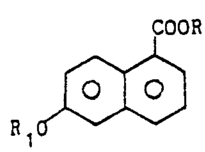

(I)

in which R = H or lower alkyl and $R_1$ = lower alkyl, which comprises reacting, in the presence of a condensation agent, furoic acid with a compound of formula :

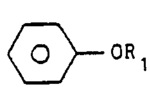

(II)

where $R_1$ has the same meaning as above, followed by acid hydrolysis, which leads to a reaction mixture comprising two compounds respectively of formula (III) and formula (IV) :

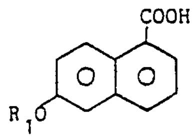 (III) 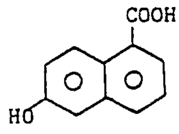 (IV)

where $R_1$ has the same meaning as before, characterized in that it comprises the action of an alkylating agent on the compounds (III) and (IV) of said mixture or on these compounds in the form of salts, this alkylating agent being chosen so as to form the compound of formula :

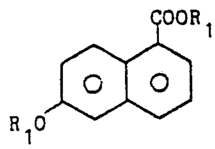

(Ia)

where $R_1$ has the same meaning as before and, if desired, hydrolysis of the ester group of the compound (Ia) so as to obtain the corresponding acid of formula :

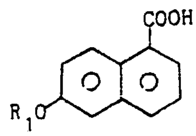

(Ib)

2. The process according to claim 1, characterized in that previous to the action of said alkylating agent on the compounds (III) and (IV), these latter are separated from the reaction mixture.

3. The process according to claim 2, characterized in that the separation is effected by extracting the reaction mixture at a pH of 7-9 by means of a basic agent in an aqueous medium.

4. The process according to claim 3, characterized in that the basic agent is an alkali metal bicarbonate.

5. The process according to any one of the preceding claims, characterized in that the alkylating agent is chosen from the group comprising the lower alkyl halides, the lower alkyl sulphates, the di(lower alkyl) sulphates, and mixtures thereof.

6. The process according to claim 5, characterized in that the alkylating agent is used in an aqueous medium or in a medium comprising water and a solvent chosen from acetone, methyl ethyl ketone, methylene chloride and 1,2-dichloroethane.

7. The process according to any one of the preceding claims, characterized in that the alkylating agent is used in the presence of a weak base.

8. The process according to any one of the preceding claims, characterized in that the condensation agent is formed by a mixture of aluminium chloride and a dehydrating acid.

9. The process according to claim 8, characterized in that the dehydrating acid is chosen from the group comprising sulphuric acid, phosphoric acid, phosphoric anhydride and mixtures thereof.

10. The process according to any one of the preceding claims, characterized in that the reaction of furoic acid with the compound (II) is carried out in the presence of a solvent chosen from the group comprising : the polyhalogenated aliphatic hydrocarbons, the polyhalogenated aromatic hydrocarbons and mixtures thereof.

11. The process according to claim 10, characterized in that the polyhalogenated aliphatic hydrocarbon is 1,1,2,2-tetrachloroethane and in that the polyhalogenated aromatic hydrocarbon is ortho dichlorobenzene.

12. The process according to any one of the preceding claims for preparing the compounds of formula (I) were $R_1$ = methyl and R = H or methyl, characterized in that the compound of formula (II) is anisole and in that the alkylating agent is a methylating agent.

13. A process for preparing a compound of formula :

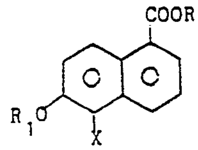

(V)

where R = H or lower alkyl, $R_1$ = lower alkyl and X = halogen, consisting in preparing the compound of formula :

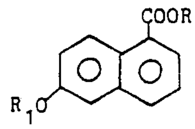

(I)

where $R_1$ and R have the same meaning as in the formula (V), from furoic acid and the compound of formula :

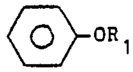

(II)

where $R_1$ = lower alkyl, then in treating the compound (I) thus obtained by means of a halogenating agent, characterized in that the compound (I) is prepared in accordance with the process according to any one of claims 1 to 12.

14. Process according to claim 13, characterized in that treatment of compound (I) with the halogenating agent is carried out in a water-halogenated aliphatic hydrocarbon medium.

## Patentansprüche

1. Verfahren zur Herstellung einer Verbindung der Formel

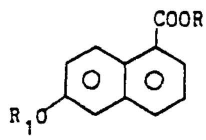

(I)

worin R = H oder niederes Alkyl und $R_1$ = niederes Alkyl, das umfaßt die Umsetzung von Furancarbonsäure mit einer Verbindung der Formel

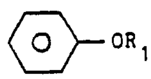

(II)

worin $R_1$ die gleiche Bedeutung wie oben angegeben hat, in Gegenwart eines Kondensationsmittels, woran sich eine saure Hydrolyse anschließt unter Bildung eines Reaktionsgemisches, das zwei Verbindungen mit dem jeweiligen Formeln (III) und (IV) umfaßt :

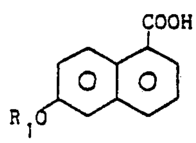

(III)

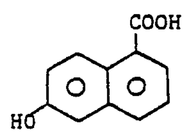

(IV)

worin $R_1$ die gleiche Bedeutung wie oben hat, dadurch gekennzeichnet, daß man außerdem auf die Verbindungen (III) und (IV) des Gemisches oder auf diese Verbindungen in Form ihrer Salze ein Alkylierungsmittel einwirken läßt, das so ausgewählt wird, daß die Verbindung der Formel gebildet wird

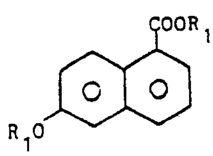

(Ia)

worin $R_1$ die gleiche Bedeutung wie oben hat, und gewünschtenfalls die Estergruppe der Verbindung (Ia) hydrolysiert unter Bildung der entsprechenden Säure der Formel

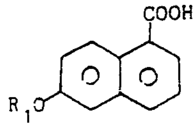

(Ib)

2. Verfahren nach Anspruch 1, dadurch gekennzeichnet, daß man vor der Einwirkung des Alkylierungsmittels auf die Verbindungen (III) und (IV) diese aus dem Reaktionsgemisch abtrennt.

3. Verfahren nach Anspruch 2, dadurch gekennzeichnet, daß die Abtrennung durch Extrahieren des Reaktionsgemisches bei einem pH-Wert von 7 bis 9 mittels eines basischen Agens im wäßrigen Milieu durchgeführt wird.

4. Verfahren nach Anspruch 3, dadurch gekennzeichnet, daß es sich bei dem basischen Agens um ein Alkalimetallhydrogencarbonat handelt.

5. Verfahren nach einem der vorhergehenden Ansprüche, dadurch gekennzeichnet, daß das Alkylierungsmittel ausgewählt wird aus der Gruppe der Niedrigalkylhalogenide, der Niedrigalkylsulfate, der Di(niedrigalkyl) sulfate und ihrer Mischungen.

6. Verfahren nach Anspruch 5, dadurch gekennzeichnet, daß das Alkylierungsmittel im wäßrigen Milieu oder in einem Milieu verwendet wird, das Wasser und ein Lösungsmittel, ausgewählt aus Aceton, Methylethylketon, Methylenchlorid und 1,2-Dichlorethan, umfaßt.

7. Verfahren nach einem der vorhergehenden Ansprüche, dadurch gekennzeichnet, daß man das Alkylierungsmittel in Gegenwart einer schwachen Base verwendet.

8. Verfahren nach einem der vorhergehenden Ansprüche, dadurch gekennzeichnet, daß das Kondensationsmittel aus einem Gemisch von Aluminiumchlorid und einer dehydratisierenden Säure besteht.

9. Verfahren nach Anspruch 8, dadurch gekennzeichnet, daß die dehydratisierende Säure ausgewählt wird aus der Gruppe Schwefelsäure, Phosphorsäure, Phosphorsäureanhydrid und ihren Mischungen.

10. Verfahren nach einem der vorhergehenden Ansprüche, dadurch gekennzeichnet, daß die Umsetzung der Furancarbonsäure mit der Verbindung (II) in Gegenwart eines Lösungsmittels durchgeführt wird, das ausgewählt wird aus der Gruppe der polyhalogenierten aliphatischen Kohlenwasserstoffe, der polyhalogenierten aromatischen Kohlenwasserstoffe und ihren Mischungen.

11. Verfahren nach Anspruch 10, dadurch gekennzeichnet, daß es sich bei dem polyhalogenierten aliphatischen Kohlenwasserstoff um 1,1,2,2-Tetrachlorethan handelt und daß es sich bei dem polyhalogenierten aromatischen Kohlenwasserstoff um o-Dichlorbenzol handelt.

12. Verfahren nach einem der vorhergehenden Ansprüche zur Herstellung von Verbindungen der Formel (I), worin $R_1$ = Methyl und R = H oder Methyl, dadurch gekennzeichnet, daß es sich bei der Verbindung der Formel (II) um Anisol und bei dem Alkylierungsmittel um ein Methylierungsmittel handelt.

13. Verfahren zur Herstellung einer Verbindung der Formel

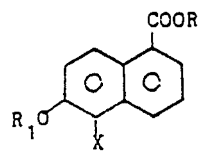

(V)

worin R = H oder niederes Alkyl, $R_1$ = niederes Alkyl und X = Halogen, das darin besteht, daß die Verbindung der Formel

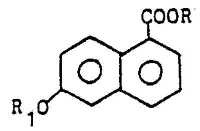

(I)

worin $R_1$ und R die gleichen Bedeutungen wie für die Formel (V) angegeben haben, hergestellt wird aus Furancarbonsäure und der Verbindung der Formel

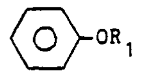

(II)

worin $R_1$ = niederes Alkyl, wonach die so erhaltene Verbindung (I) mit einem Halogenierungsmittel behandelt wird, dadurch gekennzeichnet, daß die Herstellung der Verbindung (I) nach dem Verfahren gemäß einem der Ansprüche 1 bis 12 durchgeführt wird.

14. Verfahren nach Anspruch 13, dadurch gekennzeichnet, daß die Behandlung der Verbindung (I) mit dem Halogenierungsmittel in einem Wasser/halogenierter aliphatischer Kohlenwasserstoff-Milieu durchgeführt wird.